# EUROPEAN PATENT APPLICATION

(11) **EP 3 837 982 A1**
(43) Date of publication of application: **23.06.2021**
(21) Application number: 19216801.1
(22) Date of filing: 17.12.2019
(51) Int. Cl.: A23B 7/16, A23D 7/005, C08L 91/06, C11C 3/00, C07C 45/48

(54) **WAX DISPERSIONS SUITABLE FOR THE COATING OF FOOD PRODUCTS COMPRISING KETONES WITH A MELTING POINT ABOVE 40°C**

(71) Applicant: Solvay SA, 1120 Brussels (BE)
(72) Inventor: DERRIEN, Elie, 69530 Btignais (FR); MASTROIANNI, Sergio, 69006 LYON (FR); MARION, Philippe, 69390 VERNAISON (FR)
(74) Representative: Vande Gucht, Anne

(57) **Abstract**

A wax dispersion comprising
(1) a wax comprising one ketone with a melting point above 40°C or a mixture of ketones the melting point of which or, if the mixture of ketones comprises more than one melting point, the lowest melting point of which is above 40°C,
(2) water as continuous phase, and
(3) at least one dispersing aid.

## Description

The present invention relates to wax dispersions for the coating of food products or portions thereof.

Food products such as fruits and vegetables are ready-to-eat products for which the fresh and nutritional quality should be maintained for as long as possible.

Despite this goal, a significant amount of food products, in particular fruits or vegetables is lost along the supply chain due to spoilage.

Waxy coating compositions have been used for a long period of time to improve the shelf life in particular of fruits or vegetables and are a common approach to protect fruits or vegetables e.g. during transport from the place of production to the end-consumer and to improve the shelf life of the said products.

Growing consumer requests to use natural products push formulators to replace products of fossil or animal origin by natural products.

Furthermore, there is an ongoing interest in reducing the amount of spoiled food which is lost due to lack of stability during transport or storage.

Carnauba wax, also called Brazil wax or palm wax, is a wax of the leaves of the palm *Copernicia prunifera* (Synonym: *Copernicia cerifera*), a plant native to and grown only in the northeastern Brazilian states of Piauí, Ceara, Maranhão, Bahia, and Rio Grande do Nort, is widely used for the preservation of fruits and vegetables by waxing. However, due to its limited small geographical distribution, carnauba wax suffers from supply issues which cause quality, shortage or cost risks.

US 2,383,451 relates to a process for preparing an aqueous wax emulsion suitable for the coating of fresh vegetable products which comprises preparing a hot fluid mixture of waxy material including ouricury wax and a fatty acid.

US 2,424,952 discloses a process for preparing an emulsion for application to fruits and vegetables which consists in heating candellilla wax and oleic acid, adding an aqueous solution of sodium hydroxide and adding water to the mixture.

US 3,420,790 relates to aqueous emulsions for the coating of fruits or vegetables comprising an emulsifiable polyethylene, a natural wax, an emulsifying agent and a stabilizer. Carnauba wax, candelilla wax and beeswax are used as natural waxes.

US 4,039,470 discloses compositions for applying a protective adhering film to the surface of fruits and vegetables comprising a volatile petroleum solvent, a waxy film forming material dissolved therein and a fungicidal benzimidazole.

US 4,336,273 relates to a process for treating fruit or vegetable material to minimize loss or texture during processing like canning comprising contacting the fruit or vegetable material with an organic compound selected from the group consisting of alcohols, aldehydes, amides, esters, hydrocarbons, ketones, lactones and nitriles. 4-heptanone and 5-nonanone are disclosed as ketones, which have a melting point far below 40°C.

The processes and compositions of the prior art are not fully satisfactory so that there exists an ongoing need to develop compositions suitable for the coating of fruits or vegetables.

It was thus an object of the present invention to provide compositions suitable for the protective coating of food products or portions thereof which overcome, at least partly, the disadvantages of the prior art compositions and processes.

This object is achieved with the wax dispersion of claim 1.

Preferred embodiments are set forth in the dependent claims and the detailed specification hereinafter.

Further objects of the present invention are a process for the manufacture of the wax dispersion in accordance with the present invention, a method for coating a food product with the wax dispersion in accordance with the present invention, a food composition comprising a food product and, coated on the food product or at least a portion thereof, a wax comprising one ketone with a melting point above 40°C or a mixture of ketones the melting point of which or, if the mixture of ketones comprises more than one melting point, the lowest melting point of which is above 40°C.

Finally, still another object of the present invention is the use of a wax comprising one ketone with a melting point above 40°C or a mixture of ketones the melting point of which or, if the mixture of ketones comprises more than one melting point, the lowest melting point of which is above 40°C, as a coating for food products or at least portions thereof.

Preferred embodiments are set forth in the detailed specification hereinafter and in the respective dependent claims.

The term dispersion, when used herein, is intended to cover any system in which distributed particles of one material are dispersed in a continuous phase of another material. The two phases may be in the same or different states of matter. Accordingly, the term dispersion includes heterogeneous systems comprising two immiscible liquid phases in which one is intimately distributed in the other as minute droplets and which are also commonly referred to as emulsions.

The wax dispersions in accordance with the present invention may thus be systems comprising solid particles dispersed in a continuous phase as well as systems comprising two liquids such as e.g. oil-in water emulsions.

In accordance with a preferred embodiment of the present invention, the wax dispersion is an oil-in water emulsion, optionally stabilized with dispersing aids (as hereinafter defined) to obtain emulsions stable over extended periods of time.

Component (1) of the wax dispersions in accordance with the present invention, is a wax comprising one ketone with a melting point above 40°C or a mixture of ketones the melting point of which, or, if the mixture of ketones comprises more than one melting point, the lowest melting point of which, is above 40°C.

Preferred ketones are represented by general formula R₁-C(=O)-R₂, wherein R₁ and R₂, which may be the same or different, represent a saturated or unsaturated, straight chain or branched aliphatic radical with of from 7 to 23, preferably from 7 to 19 and even more preferred from 7 to 18 carbon atoms, preferably a saturated aliphatic group and more preferably an alkyl group of from 7 to 23, preferably from 7 to 19 and even more preferred from 7 to 17 carbon atoms.

Examples of suitable radicals R₁ and R₂ are octyl (C₈), nonyl (Cg), decyl (C₁₀), undecyl (C₁₁), dodecyl (C₁₂), tridecyl (C₁₃) tetradecyl (C₁₄), hexadecyl (C₁₆), heptadecyl (C₁₇), octadecyl (C₁₈) and eicosyl (C₂₀), to name only some examples.

In accordance with a preferred embodiment the ketone comprised in the wax dispersions in accordance with the present invention is obtained by decarboxylative ketonization of a fatty acid, a fatty acid derivative or a mixture thereof in the liquid phase with a metal or a metal compound as catalyst.

The fatty acid has generally from 4 to 28 carbon atoms, preferably from 8 to 24 carbon atoms.

More preferably, the fatty acid is selected from the group consisting of isostearic acids, caprylic acid, capric acid, lauric acid, myristic acid, palmitic acid, stearic acid, arachidic acid, behenic acid, lignoceric acid, cerotic acid and mixtures thereof.

The term "fatty acid derivative" refers to an anhydride made by the condensation of 2 fatty acids or to an ester made by the condensation of a fatty acid with an alcohol.

Suitable fatty acid derivatives are esters and anhydrides of fatty acids, but the use of free fatty acids as such is generally preferred. The esters or anhydrides in the course of the reaction are converted to the acids which then react with the metal or the metal compound.

The fatty acids or fatty acid derivatives can be used in the form of so called fatty acid or fatty acid derivatives cuts which may be obtained by the hydrolysis or alcoholysis of different natural fats and oils. Accordingly these cuts may contain various amounts of different linear fatty acids or linear fatty acid derivatives with different chain lengths. Just by way of examples fatty acid cuts obtained from coconut oil and comprising mainly C₁₂-C₁₈ fatty acids may be mentioned here. The skilled person is well aware of other fatty acid cuts obtainable from various sources and will select the best suitable starting materials based on the desired ketones.

In accordance with a preferred embodiment, fatty acids having 12 carbon atoms or less, preferably from 8 to 12 carbon atoms or derivatives of such acids (esters or anhydrides) constitute at least 10 mol% and preferably at least 15 mol% of the entire molar amount of the fatty acid mixture or fatty acids derivatives mixture used as starting material for the manufacture of the ketones in the wax (1) of the dispersion in accordance with the present invention. These acids lead to ketones having a total carbon number of 23 or less which have proved to be advantageous in a number of applications. There is no specific upper limit for the amount of these fatty acids or fatty acid derivatives of acids having 12 carbon atoms or less, i.e. the starting material may also entirely consist of such fatty acids or fatty acid derivatives.

The fatty acids may comprise one or more double bonds in the chain such as oleic acid, linoleic acid, linolenic acid, erucic acid, palmitoleic acid or mixtures thereof.

When starting from a single fatty acid, a symmetrical ketone is obtained as the reaction product; when starting from a cut of fatty acids as described above all the ketones formed by the combination of the different alkyl groups of the starting acids are obtained and the distribution of the different mixed ketones generally follows a statistical binomial law. The reaction equation can be summarized as follows:

Rₙ-COOH + Rₘ-COOH → Rₙ-C(=O)-Rₘ + CO₂ + H₂O

wherein Rₙ and Rₘ represent the aliphatic groups of the fatty acids present in the cut. It is well apparent that e.g. if three different acids are present, a total of six different ketones may be formed; three symmetrical ketones wherein Rₙ and Rₘ are identical and three unsymmetrical ketones with different groups Rₙ and Rₘ.

The conversion of fatty acids into respective ketones by decarboxylative ketonization is a well known process which is also commercially used and has been described in the literature. Suitable processes for the manufacture of suitable ketones are e.g. disclosed in EP 3 292 097, WO 2018/087179 and WO 2018/033607 to which reference is made here for further details.

The processes disclosed in EP 3 292 097, WO 2018/087179 and WO 2018/033607 offer an easy access to internal ketones which can be used as ketones in the wax of the wax dispersions in accordance with the present invention.

The ketones may be separated from the reaction mixture by convenient and economic processes and the catalytic material can be used for several catalytic cycles without significant deterioration of catalytic activity.

The wax (1) in the wax dispersion of the present invention may comprise a single ketone or a mixture of ketones. The ketones may be symmetric or unsymmetric.

The weight percentage of the wax in the wax dispersion of the present invention is not subject to particular limitations and the skilled person will adjust the content based on the need of the specific application based on his professional knowledge.

The content of the wax (1) in the wax dispersion in accordance with the present invention is preferably in the range from 2 to 40, more preferably in the range from 5 to 30 and in particular in the range from 7 to 25 wt%, based on the weight of the wax dispersion.

The wax (1) in the wax dispersions in accordance with the present invention preferably consists essentially of a ketone or a ketone mixture, and even more preferably consists of a ketone or a mixture of ketones as described above.

As component (2), the wax dispersion in accordance with the present invention comprises water in an amount so that water forms the continuous phase of the dispersion. The water content of the ready-to-use dispersion is adjusted so that the dispersion can be suitably coated onto the food products or portions thereof, preferably fruits or vegetables or portions thereof.

In some cases, it has proven to be advantageous to adjust the viscosity of the wax dispersions in accordance with the present invention to a value similar to the viscosity of Carnauba wax dispersions used for coating food products or portions thereof, in particular fruits and vegetables or portions thereof.

The water content in the wax dispersion of the present invention is not subject to particular limitations but is preferably in the range from 35 to 85, more preferably in the range from 45 to 80 wt%, based on the weight of the wax dispersion.

As component (3), the wax dispersion in accordance with the present invention comprises at least one dispersing aid.

The term dispersing aid, when used herein, refers to dispersing agents as well as to dispersion stabilizers.

A dispersant or dispersing agent for the purpose of the present invention is a surface active substance to improve the separation of the particles and to prevent settling or clumping of the composition. A solid material dispersed in a liquid often requires an additive to make the dispersion process easier and more stable - this is the role of a dispersing agent or dispersant. Dispersion stabilizers are substances which make it possible to maintain the physico-chemical state of a dispersion, e.g. substances which enable the maintenance of a homogenous dispersion of two or more immiscible substances. Dispersants may also act as dispersion stabilizers and vice versa but respective products do not necessarily exhibit such dual function.

Suitable dispersing aids are known to the skilled person and have been described in the literature. Based on his professional knowledge the skilled person will select suitable materials to be used as dispersing aids in the dispersions in accordance with the present invention so that no further details need to be given here. The amount and nature of the component (3) can be used to adjust the stability and viscosity of the dispersion and to adjust the particle size of the dispersed phase in the dispersion in accordance with the present invention. While the content of component (3) in the wax dispersion of the present invention is thus not subject to particular limitations, the content of component (3) is preferably in the range from 0.1 to 20, preferably from 0.3 to 18 and particularly preferred in the range from 0.5 to 15 wt% based on the weight of the wax dispersion.

An exemplary group of compounds suitable for use in the component (3) consists of surfactants.

For the purpose of the present invention, the term "surfactant" means a compound that, when present in water, lowers the surface tension of water. Surfactants may notably act as dispersants.

Surfactants can be generally grouped into ionic and non-ionic representatives. For the formulations according to the invention, non-ionic and anionic surfactants are generally preferred.

Exemplary representatives of non ionic surfactants are fatty (C₄-C₂₈) acid ethoxylates and propoxylates, fatty alcohol ethoxylates and propoxylates (such as 10 E.O. ethoxylated cetyl alcohol), nonylphenol or octylphenol ethoxylates and propoxylates [such as 10 E.O. 4-(1,1,3,3-tetramethylbutyl)phenyl-polyethylene glycol], sorbitan fatty acid esters (such as sorbitan monooleate), sorbitan fatty acid esters ethoxylates and propoxylates (such as Tween® 80 ethoxylated sorbitan monooleate, commercially available from CRODA America, Inc.) and poly(alkylene oxide)s.

Poly(alkylene oxide)s are polymers essentially all or all the repeating units of which comply with general formula -p-O-wherein p represents a divalent alkylene group having from 2 to 10 carbon atoms. Such poly(alkylene oxide)s may be terminated by a hydroxyl group. Particularly suitable poly(alkylene oxide)s are those wherein p has from 2 to 4 carbon atoms, preferably from 2 to 3 carbon atoms, more preferably 2 carbon atoms. The poly(alkylene oxide)s may be either linear or branched. Linear poly(alkylene oxides) are generally preferred. Specific examples of suitable poly(alkylene oxide)s include polyoxyalkylene polyols, such as polyoxyethylene glycol (also known as poly(ethylene glycol) or poly(ethylene oxide), polyoxyethylene triol, polyoxyethylene tetraol, polyoxypropylene glycol (also commonly referred to as poly(propylene glycol) or poly(propylene oxide), polyoxypropylene triol, polyoxypropylene tetraol, polyoxybutylene glycol, polyoxypentane glycol, polyoxyhexane glycol, polyoxyheptane glycol, and polyoxyoctane glycol. These polymers may be used either individually or in combinations of two or more; for example, it can be cited random copolymers of ethylene oxide and propylene oxide, and poly(ethylene oxide)-poly(propylene oxide) block copolymers. The molecular weight of the poly(alkylene oxide)s may cover a wide range. In certain cases poly(alkylene oxide)s having a number average or weight average molecular weight of at least 20,000, preferably at least 200,000 and even more preferably at least 1,000,000 are advantageous. In other cases average molecular weights of at most 20,000, preferably at most 10,000 and even more preferably at most 1000 are useful. The molecular weight of the suitable poly(alkylene oxides) may also be optimized in view of the specific application case. For example, a methoxy-terminated poly(ethylene oxide) having a number average or a weight average molecular weight of at most 2,000 may be used. Copolymers comprising oxyethylene and oxypropylene units in random or block distribution may also be suitable and respective products are commercially available under the tradename Pluronics® from BASF, such as Pluronics® 68 copolymer.

Exemplary representatives of anionic surfactants are alkali metal or ammonium carboxylates (such as ammonium myristate and sodium oleate), alkali metal or ammonium alkyl sulfates (such as sodium lauryl sulfate), alkali metal or ammonium alkyl sulfonates (such as sodium lauryl sulfonate), alkali metal or ammonium alkyl aryl sulfonates (such as sodium dodecylbenzene sulfonate), alkali metal or ammonium sulfosuccinates, phospholipids and sulfolipids.

Oleic acid and its derivatives may be mentioned as a further example of a compound suitable for use as the component (3) in the dispersion in accordance with the present invention. Oleic acid may also be useful to adjust the viscosity of the wax dispersion in accordance with the present invention.

The pH value of the dispersion may be adjusted with suitable additives such as ammonium salts or alkali metal hydroxides or mixtures thereof. The amount of the various dispersing aids which may be present as component (3) is not subject to particular limitations and the skilled person will select suitable amounts based on his professional knowledge and the specific application situation.

Typical contents of component (3) are as disclosed hereinbefore.

In accordance with an embodiment of the present invention, the wax (1) comprises in addition to a ketone or a ketone mixture as described above at least one other wax component which may be a natural wax such as carnauba wax or candelilla wax to name only two examples. Other examples of wax components susceptible of being comprised in the wax (1) in addition to the ketone or ketone mixture are polyolefin wax, Ozokerite, microcristalline wax, paraffin wax, Fischer-Tropsch wax, montan wax, Ceresin, beeswax, jojoba wax, rice bran wax, sugarcane wax, japan wax, tea wax, sunflower wax, berry wax, myrica fruit wax, laurel wax, lanolin, spermaceti, fatty ester wax and fatty acid wax.

The presence of one or more wax components other than the ketone or ketone mixture in the wax (1) comprised in the wax dispersion according to the present invention allows tailoring the coating with regard to specific properties.

If the wax (1) comprises, in addition to the ketone or ketone mixture as described above, at least one other wax component, the weight ratio of the other wax component to the ketone or mixture of ketones is not subject to particular limitations and is preferably in the range of from 5:95 to 95:5, more preferably in the range of from 10:90 to 90:10.

The wax dispersions of the present invention are suitable for the coating of any food product or portion thereof.

The properties of the dispersion can be adjusted to the specific food product to be coated.

Generally any food product may be coated with the wax dispersion in accordance with the present invention.The term food product for the purpose of the present invention is intended to cover any fresh or processed product suitable for consumption by humans or animals. Just by way of example, fruits, vegetables, cheese and other products derived from or based on milk, cocoa and chocolate products, sweets, nuts and processed nuts, coffee beans, cereals and chewing gum may be mentioned here.

A preferred group of food products are fruits or vegetables.

Preferred fruits and vegetables are selected from the group consisting of apples, avocados, bananas, bell peppers, Chili peppers, citrus fruits, cherries, cucumbers, eggplant, grapefruits, mangoes, pineapples melons, papayas, parsnips, passion fruit, pomegranate, eggfruit, peaches, pears, plums, squash, swedes (or rutabagas), potatoes, parsnips, squashes, cassavas, sweet potatoes, tangerines, tomatoes and turnips, of which apples, citrus fruits and mangoes may be particularly preferably coated with the dispersion of the present invention.

Another embodiment of the present invention relates to a method for preparing a wax dispersion in accordance with the present invention comprising the steps of (i) mixing a wax comprising one ketone with a melting point above 40°C or a mixture of ketones the melting point of which or, if the mixture of ketones comprises more than one melting point, the lowest melting point of which is above 40°C, (ii) water in an amount to form the continuous phase of the wax dispersion, and (iii) at least one dispersing aid, under stirring and heating the mixture to a temperature not exceeding 100°C to obtain a dispersion of the wax in the continuous aqueous phase.

A further embodiment of the present invention relates to a method for coating a food product, in particular for the coating of fruits or vegetables, comprising applying on the whole food product or a portion thereof a wax dispersion in accordance with the present invention.

The coating may be applied by any known method for applying liquid coatings onto solid surfaces. Spraying may be mentioned as particularly preferred.

Preferably the method for coating a food product or at least a portion thereof in accordance with the present invention comprises an additional step of drying the dispersion coated on the food products or at least a portion thereof to remove water from the coating and, optionally, brushing or polishing the coated food product or a coated portion thereof.

Preferably the method is applied to fruits or vegetables or portions thereof selected from the group consisting of apples, avocados, bananas, bell peppers, cassava, citrus fruits, cherries, chili peppers, cucumbers, eggplant, grapefruits, mangoes, melons, papayas, parsnips, passion fruit, peaches, pears, plums, squash, swedes (or rutabagas), sweet potatoes, tangerines, tomatoes and turnips, even more preferably to apples, oranges and mangoes.

Still another embodiment of the present invention relates to a food composition comprising (A) a food product and, coated on the whole food product or at least on a portion thereof, (B) a wax comprising one ketone with a melting point above 40°C or a mixture of ketones the melting point of which or, if the mixture of ketones comprises more than one melting point, the lowest melting point of which is above 40°C.

The wax (B) comprised in the food composition is the wax (1) comprised in the wax dispersion in accordance with the present invention.

The ketone(s) of the wax (B) is (are) the ketone(s) described before for the wax (1) of the dispersion in accordance with the present invention. Reference is made to the preceding description of the wax (1) and ketones here to avoid duplications.

The food component of the food composition in accordance with the present invention may be any food product in natural form or in another physical status where coating can improve the shelf life of the food product.

Preferably the food component of the food composition in accordance with the present invention is selected from the group consisting of apples, avocado, bananas, bell peppers, cassava, citrus fruits, cherries, chili peppers, cucumbers, eggplant, grapefruits, mangoes, melons, papayas, parsnips, passion fruit, peaches, pears, plums, squash, swedes (or rutabagas), sweet potatoes, tangerines, tomatoes and turnips, even more preferably from apples, oranges and mangoes.

Finally, an embodiment of the present invention relates to the use of a wax comprising one ketone with a melting point above 40°C or a mixture of ketones the melting point of which or, if the mixture of ketones comprises more than one melting point, the lowest melting point of which is above 40°C as a coating for food products, preferably fruits or vegetables, or at least portions of food products, preferably to improve the shelf life of the food product.

The term "shelf life", for the purpose of the present invention, is intended to denote any improvement in the storage stability, optical appearance or physical properties at extended storage times.

The wax may be preferably applied to fruits or vegetables selected from the group consisting of apples, avocado, bananas, bell peppers, cassava, citrus fruits, cherries, chili peppers, cucumbers, eggplant, grapefruits, mangoes, melons, papayas, parsnips, passion fruit, peaches, pears, plums, squash, swedes (or rutabagas), sweet potatoes, tangerines, tomatoes and turnips.

The present invention provides an improvement over the prior art, which improvement may be a longer shelf life, a stability of the physical properties over longer periods of time or an improvement in the sensory, color or texture appearance of the food product after extended storage times.

The present invention can thus contribute to reduce the amount of lost food products which is economically and ecologically desirable.

The following examples show the effectiveness of the present invention.

### Example 1 - Synthesis of 12-tricosanone (dialiphatic ketone of lauric acid)

The reaction was carried under argon in a round bottom flask equipped with mechanical stirring, Dean Stark apparatus and an addition funnel. In the reactor, 700 mg of iron powder were dispensed and 20g of lauric acid was introduced into the addition funnel.

A first partial amount of 5g of acid was added into the reactor and the temperature was brought to 250°C. The mixture was stirred at this temperature for 30 minutes during which the color of the media changed to black and H₂ gas was released.

Then the temperature was raised to 300°C, the mixture was stirred during 1h30 and the remaining amount of lauric acid (15 grams) was slowly added into the reactor during 4h30min at a flow rate which allowed keeping concentration of lauric acid in the reaction media very low (no accumulation of free acid in solution).

At the end of the reaction, the addition funnel was replaced by a distillation apparatus and the products were distilled off at 290°C-340°C under 5 kPa pressure.

Then the distillation apparatus was replaced by the addition funnel containing a new batch of 20g of fatty acids and the operations described above were repeated for another cycle. No additional amount of iron was needed. The residue in the flask remaining after distillation was efficient to convert the next batch of acids.

Overall 4 cycles were carried out without any loss of performances reducing thereby the concentration of iron to less than 1 wt% relative to fatty acids amount converted.

18-pentatriacontanone (dialiphatic ketone of stearic acid) was prepared in an analogous manner.

A C15-C35 ketone mixture (hereinafter referred to as IK C15-C35) was obtained from a respective C8-C18 fatty acid cut in an analogous manner.

### Example 2 - Preparation of dispersions

40 g of each of the dialiphatic ketones obtained in Example 1, 175 g of water, 28 ml of 1% NH₄OH and 5 ml of 0.1N NaoH, 15 g of oleic acid and 1 ml of polyethylene glycol (PEG, number molecular weight 4000 g/mol, PEG-4000) were mixed under stirrring and heated to a temperature of 93°C. For comparative purposes a dispersion was prepared in which the dialiphatic ketone wax was replaced by carnauba wax.

While the dispersions with carnauba wax and 18-pentatriacontanone were flowing directly (i.e. had the correct viscosity for being spreaded on the fruits), the dispersions with 12-tricosanone and IK C15-C35 had to be futher diluted with water to adjust the suitable viscosity matching the viscosity of carnauba wax and 18-pentatriacontanone dispersions.

### Example 3 - Coating of fruits

The fruits were washed and sanitized before applying the coating. The coating was carried out using a pulverizer (Tander, model TPTE652). After that, the samples were kept overnight for drying the emulsion on fruits and followed by polishing with a brush and a multi-purpose clean cloth. Then, the samples were immediately stored in incubators kept at 25 °C and 85% RH (relative humidity). About 30 samples of orange and apples, and about 21 samples of mangoes were used for for each of the five storage conditions
i) uncoated
ii) coated with Carnauba wax
iii) coated with 18-pentatriacontanone
iv) coated with 12-tricosanone
v) coated with IK C15-C35

Additionally, 5 samples of each fruit were selected for monitoring weight loss.

Shelf life was evaluated by performing physicochemical analyses at a predetermined time for each fruit. For each analysis, three fruits of each storage condition were used. First, photos were taken for peel color determination. So, half of the fruits were used for pulp color, and texture determination and the other half was peeled and ground in a household blender (Philco, Brazil) for determining aw, moisture, pH, acidity, and soluble solids. All the measurements were made in triplicate for each sample.

The fruits were analyzed visually as to color, brightness, and firmness. The parameters were compared on a grade of 0 to 5, in which the maximum value (5) was related to the control of the first day (day 0).

### Results

### Apples

The literature describes apple shelf-life to be around 25 days. Apple properties were evaluated every four days until reaching the expected shelf life and after 49 days of storage.

Tricosanone and IK C15-C35 wax dispersions were more efficient in preserving the color, brightness and texture (especially firmness, elongation of the peel and crunchiness of the pulp) of the apples compared to the comparative coating with carnauba wax.

For moisture, soluble solids, water activity, pH and acidity value the results were comparable for the dialiphatic ketone waxes to the Carnauba wax.

### Oranges

The literature describes orange shelf-life to be around 40 days. Orange properties were evaluated every seven days until reaching the expected shelf life and after 65 days of storage.

In the sensory analysis the IK C15-C35 and Tricosanone waxes were more efficient in preserving the oranges sensory characteristics than carnauba wax. IK C15-C35 wax preserved the green color of oranges peel until 65 days of storage while oranges coated with other waxes became yellow earlier. 12-Tricosanone wax coated fruits spoiled after 42 days of storage while the other waxes did not show significant differences to the uncoated fruits. The same was true for pulp color parameters.

Until day 42 (i.e. until the end of the expected shelf life) all dialiphatic ketone waxes were more efficient in preserving the texture of the peel of the fruit than control.

The oranges coated with IK C15-C35 wax was most efficient in preserving the crunchiness of the orange pulp.

For weight loss, moisture, soluble solids, water activity, pH and acidity, the results of coated und uncoated oranges showed no significant difference.

### Mangoes

Mango shelf life is expected to be 2-3 weeks. In the course of the present evaluation, mangoes spoiled after two weeks. They were evaluated every three days until the end of the shelf life.

18-pentatriacontanone and 12-tricosanone coated fruits in the sensory analysis were comparable to Carnauba wax, while IK C15-C35 wax was somewhat less effective in preserving color, brightness and firmness.

The IK C15-C35 wax coated fruits showed a delayed color change which is an important aspect for customers.

Carnauba and IK C15-C35 wax coated fruits showed less weight loss than the other waxes. Moisture, pH, soluble solids, water activity and acidity were comparable in the uncoated fruits and the coated fruits.

## Claims

1. A wax dispersion comprising
(1) a wax comprising one ketone with a melting point above 40°C or a mixture of ketones the melting point of which or, if the mixture of ketones comprises more than one melting point, the lowest melting point of which is above 40°C,
(2) water as continuous phase, and
(3) at least one dispersing aid.

2. The wax dispersion of claim 1 wherein the wax comprises a ketone of formula R₁-C(=O)-R₂ or a mixture of ketones of formula R₁-C(=O)-R₂ wherein R₁ and R₂, which may be the same or different, represent a saturated or unsaturated, straight chain or branched aliphatic radical with of from 7 to 24 carbon atoms.

3. The wax dispersion of claim 12 wherein R₁ and R₂, independent of each other, represent an alkyl group having 7 to 24 carbon atoms.

4. The wax dispersion of any of claims 1 to 3 wherein the ketone or the mixture of ketones comprised in the wax (1) is obtained by decarboxylative ketonization of a fatty acid, a fatty acid derivative or a mixture thereof in the liquid phase with a metal or a metal compound as catalyst.

5. The wax dispersion of claim 4 wherein the fatty acid is selected from isostearic acids, caprylic acid, capric acid, lauric acid, myristic acid, palmitic acid, stearic acid, arachidic acid, behenic acid, lignoceric acid, cerotic acid and mixtures thereof.

6. A method for preparing a wax dispersion in accordance with any of claims 1 to 5 comprising the steps of (i) mixing a wax comprising one ketone with a melting point above 40°C or a mixture of ketones the melting point of which or , if the mixture of ketones comprises more than one melting point, the lowest melting point of which is above 40°C, (ii) water in an amount to form the continuous phase of the wax dispersion and (iii) at least one dispersing aid, under stirring and heating the mixture to a temperature not exceeding 100°C to obtain a dispersion of the wax in the continuous aqueous phase.

7. A method for coating a food product comprising applying on the whole food product or a portion thereof the wax dispersion in accordance with any of claims 1 to 5.

8. The method of claim 7 comprising the additional step of drying the dispersion coated on the food product or at least a portion thereof to remove water from the coating and, optionally, brushing or polishing the coated fruit or vegetable or a coated portion thereof.

9. The method of claim 7 or 8 wherein the food product is a fruit or vegetable, said fruit or vegetable being selected from the group consisting of apples, avocados, bananas, bell peppers, Chili peppers, citrus fruits, cherries, cucumbers, eggplant, grapefruits, mangoes, pineapples, melons, papayas, parsnips, passion fruit, pomegranate, eggfruit, peaches, pears, plums, squash, swedes (or rutabagas), potatoes, parsnips, squashes, cassavas, sweet potatoes, tangerines, tomatoes and turnips.

10. A food composition comprising (A) a food product and, coated on the whole food product or at least on a portion thereof, (B) a wax comprising one ketone with a melting point above 40°C or a mixture of ketones the melting point of which or, if the mixture of ketones comprises more than one melting point, the lowest melting point of which is above 40°C.

11. The food composition of claim 10 wherein the food product is a fruit or vegetable.

12. The food composition of claim 11 wherein the fruit or vegetable is selected from the group consisting of apples, avocado, bananas, bell peppers, cassava, citrus fruits, cherries, chili peppers, cucumbers, eggplant, grapefruits, mangoes, pineapples, melons, papayas, parsnips, passion fruit, pomegranate, eggfruit, peaches, pears, plums, squash, swedes (or rutabagas), potatoes, sweet potatoes, parsnips, squashes, cassavas, tangerines, tomatoes and turnips.

13. Use of a wax comprising one ketone with a melting point above 40°C or a mixture of ketones the melting point of which or, if the mixture of ketones comprises more than one melting point, the lowest melting point of which is above 40°C as a coating for a food product or at least a portion of a food product.

14. Use in accordance with claim 13 to improve the shelf life of said food product or portion thereof.

15. Use in accordance with claim 13 or 14 wherein the fruit or vegetable is selected from the group consisting of apples, avocados, bananas, bell peppers, Chili peppers, citrus fruits, cherries, cucumbers, eggplant, grapefruits, mangoes, pineapples, melons, papayas, parsnips, passion fruit, pomegranate, eggfruit, peaches, pears, plums, squash, swedes (or rutabagas), potatoes, parsnips, squashes, cassavas, sweet potatoes, tangerines, tomatoes and turnips.
